# EUROPÄISCHE PATENTSCHRIFT

(11) **EP 2 711 041 B1**
(45) Veröffentlichungstag und Bekanntmachung des Hinweises auf die Patenterteilung: **10.06.2015**
(21) Anmeldenummer: 13189588.0
(22) Anmeldetag: 03.11.2007
(51) Int. Cl.: A61M 16/08

(54) **Atemschlauch-Anschlussvorrichtung**
Breathing hose connecting device
Dispositif de raccordement de tuyau respiratoire

(30) Priorität: 14.11.2006 DE 102006053857
(43) Veröffentlichungstag der Anmeldung: 26.03.2014
(62) Teilanmeldung aus: 07817766.4
(73) Patentinhaber: Dräger Medical GmbH, 23558 Lübeck (DE)
(72) Erfinder: Tappehorn, Ludger, 23560 Lübeck (DE); Wotha, Gerd, 23626 Warnsdorf (DE); Schermeier, Olaf, 23560 Lübeck (DE); Abraham, Klaus, 23560 Lübeck (DE)
(74) Vertreter: UEXKÜLL & STOLBERG

(56) Entgegenhaltungen:
- EP-A1- 1 731 089
- EP-A2- 1 800 705
- US-A1- 2003 230 307
- US-A1- 2003 236 015
- US-A1- 2004 127 937

## Beschreibung

Die Erfindung betrifft eine Atemschlauch - Anschlussvorrichtung zur Verbindung eines Atemschlauches mit einer Atemvorrichtung mit den Merkmalen von Anspruch 1.

Atemschlauch - Anschlussvorrichtungen dienen in bekannter Weise der Verbindung eines Atemschlauches oder eines Atemschlauchsystems, im Folgenden der Einfachheit halber mit Atemschlauch bezeichnet, mit einer Atemvorrichtung. Eine Atemvorrichtung kann ein Anästhesiegerät oder ein Beatmungsgerät umfassen. Die Atemschlauch - Anschlussvorrichtungen unterscheiden sich hinsichtlich des Durchmessers eines Atemgasdurchgangskanals. So weisen beispielsweise Atemschlauch-Anschlussvorrichtungen für den neonatalen Einsatz geringere Durchmesser auf, im Vergleich zu den Atemschlauch -Anschlussvorrichtungen zur Verbindung eines Atemschlauches an eine Atemvorrichtung für Erwachsene. Die Atemschlauch - Anschlussvorrichtungen verfügen dabei entweder über einen männlichen oder einen weiblichen Anschluss. Ein dazu komplementärer Anschluss ist jeweils an der Atemvorrichtung zu finden. Üblicherweise sind Atemschlauch - Anschlussvorrichtungen für eine Verbindung mit einer neonatalen Atemvorrichtung mit einem weiblichen Anschluss ausgeführt, wohingegen Atemschlauch - Anschlussvorrichtungen für eine Verbindung mit einer Atemvorrichtung für Erwachsene mit einem männlichen oder weiblichen Anschluss ausgeführt sein können. Demgegenüber sind die Anschlüsse an der neonatalen Atemvorrichtung üblicherweise als männlicher Anschluss und an der Atemvorrichtung für Erwachsene als Kombination von männlichem und weiblichem Anschluss ausgeführt.

Es existiert für die Atemschlauch - Anschlussvorrichtungen ein gängiger Standard von 11, 15, und 22 Millimeter Durchmesser des Atemgasdurchgangskanals. Dabei weisen Atemschlauch - Anschlussvorrichtungen für den neonatalen Einsatz einen Durchmesser des Atemgasdurchgangskanals von 11 Millimetern auf, wohingegen Atemschlauch - Anschlussvorrichtungen für Atemvorrichtungen für Erwachsene einen Durchmesser des Atemgasdurchgangskanals von 15 oder 22 Millimetern aufweisen können. Beispiele sind bekannt durch US 2003/236015, US 2003/230307, EP 1731089 und EP 1949930.

Die Atemvorrichtungen für Erwachsene verfügen im Allgemeinen über einen Atemmodus für Neonaten. Soll nun mit einer Atemvorrichtung für Erwachsene ein neonataler Patient beatmet werden, ist es wünschenswert, dass sich die Atemschlauch - Anschlussvorrichtung des neonatalen Atemschlauches, die für einen Anschluss an eine neonatale Atemvorrichtung konzipiert ist, sowohl mit der neonatalen Atemvorrichtung als auch mit der Atemvorrichtung für Erwachsene verbinden lässt. Darüber hinaus sollte die Atemschlauch - Anschlussvorrichtung einfach und kostengünstig herzustellen sein.

Die Aufgabe der Erfindung besteht demnach in der Bereitstellung einer einfach herzustellenden Atemschlauch - Anschlussvorrichtung, die das vorstehend beschriebene Problem beseitigt, sowie in einem Verfahren zur Herstellung einer derartigen Atemschlauch - Anschlussvorrichtung.

Diese Aufgabe wird erfindungsgemäss durch eine Atemschlauch - Anschlussvorrichtung zur Verbindung eines Atemschlauches mit einer Atemvorrichtung, bestehend aus einem Grundkörper, mit einem in dem Grundkörper ausgebildeten Atemgasdurchgangskanal und einem Hüllkörper gelöst, wobei der Hüllkörper mit dem Grundkörper formschlüssig in Eingriff bringbar ist, und dabei zumindest einen Teil des Grundkörpers koaxialförmig umschließt. Die Idee der Erfindung ist es, einen Grundkörper mit einem, dem Durchmesser des neonatalen Atemschlauches entsprechenden Atemgasdurchgangskanal und einen, in den Abmessungen dem Anschluss an die jeweilige Atemvorrichtung für Erwachsene entsprechend ausgebildeten Hüllkörper bereitzustellen, wobei der Grundkörper mit dem, für die entsprechende Atemvorrichtung für Erwachsene, vorgesehenen Hüllkörper kombinierbar ist und in der Kombination der Grundkörper und der Hüllkörper in formschlüssigen Eingriff gebracht sind, so dass ein Anschluss eines neonatalen Atemschlauches mittels der erfindungsgemässen Atemschlauch - Anschlussvorrichtung sowohl an eine neonatale Atemvorrichtung, als auch an eine Atemvorrichtung für Erwachsene erfolgen kann.

Vorteilhafte Ausführungen und Weiterbildungen der Erfindung sind in den Unteransprüchen angegeben.

Die mit der Erfindung erzielten Vorteile bestehen insbesondere darin, dass die erfindungsgemäße Atemschlauch - Anschlussvorrichtung sehr kostengünstig herstellbar ist. Es ist dabei ein Grundkörper herstellbar, der mit einem gemäß der jeweiligen Atemvorrichtung für Erwachsene entsprechenden Hüllkörper derart kombinierbar und in formschlüssigen Eingriff gebracht ist, dass die Atemschlauch - Anschlussvorrichtung sowohl an die neonatale Atemvorrichtung als auch an die Atemvorrichtung für Erwachsene anschließbar ist. Ein jeweils gleicher Grundkörper kann also mit einem Hüllkörper, der einen zum Anschluss an die entsprechende Atemvorrichtung für Erwachsene erforderlichen Durchmesser aufweist, kombiniert werden. Die erfindungsgemässe Atemschlauch - Anschlussvorrichtung ermöglicht somit eine Verbindung eines Atemschlauches sowohl mit einer ersten, als auch mit einer zweiten Atemvorrichtung, wobei die erste und die zweite Atemvorrichtung eine unterschiedliche Anschlussstruktur aufweist. Die erste Atemvorrichtung ist dabei eine neonatale Atemvorrichtung und die zweite Atemvorrichtung eine Atemvorrichtung für Erwachsene. Die Atemvorrichtung für Erwachsene weist zudem einen Atemmodus zur Beatmung von Neonaten auf.

Der Aufbau der erfindungsgemässen Atemschlauch - Anschlussvorrichtung ist derart gestaltet, dass sowohl die Außenfläche des Grundkörpers als auch die Innenwandung des Hüllkörpers vorzugsweise zylindrisch ausgebildet sind. Diese Formgestaltung ermöglicht eine stabile Verbindung von Grund- und Hüllkörper. Ein Formschluss von Grund- und Hüllkörper erfolgt vorzugsweise durch einen Eingriff einer an der Außenfläche des Grundkörpers vorgesehenen Umfangswulst in eine komplementäre, an der Innenwandung des Hüllkörpers vorgesehene Umfangsnut. Um nach dem formschlüssigen Eingreifen von Grund- und Hüllkörper ein Verdrehen des Hüllkörpers in Bezug zu dem Grundkörper zu verhindern, insbesondere bei einem Verbinden oder Lösen der Atemschlauch - Anschlussvorrichtung mit oder von der Atemvorrichtung, sind in einer weiteren Ausführung der erfindungsgemässen Vorrichtung Mittel vorgesehen, die ein Verdrehen verhindern. Vorzugsweise weist dafür der Grundkörper eine senkrecht zu der Außenfläche des Grundkörpers gerichtete Nase auf und der Hüllkörper eine komplementäre, senkrecht zu seiner Innenwandung gerichtete Aussparung. Die Nase der Außenfläche des Grundkörpers tritt bei Formschluss mit der Aussparung der Innenwandung des Hüllkörpers in Eingriff. Ein Material des Grund- und Hüllkörpers besteht vorzugsweise aus Polypropylen. Polypropylen ist in bekannter Weise ein elastisches Material, was den Vorgang des Formschlusses von Grund- und Hüllkörpers begünstigt. Zudem verläuft für eine optimale Konnektierung der Atemschlauch - Anschlussvorrichtung mit einer Atemvorrichtung der zu einer Atemvorrichtung gerichtete Teil des Atemgasdurchgangskanals des Grundkörpers und die Außenfläche des Hüllkörpers konusförmig.

In einer besonders bevorzugten Ausführung der Atemschlauch - Anschlussvorrichtung ist ein Transponder zwischen Grundkörper und Hüllkörper vorgesehen. Der Transponder wird in diesem Zusammenhang als Element zur drahtlosen Sendung und zum Empfang von Daten verstanden, das zudem mit einer Funktion zur Speicherung der Daten ausgestattet ist. Da Transponder eine hohe Wärmeempfindlichkeit aufweisen, ist ein Einbringen in den Grund- oder Hüllkörper während des Spritzgussverfahrens ohne eine Zerstörung seiner Funktionen nahezu unmöglich. Eine Positionierung des Transponders auf der Außenfläche der Atemschlauch - Anschlussvorrichtung, beispielsweise durch Aufkleben, kann einerseits Beschädigungen des Transponders beim Verbinden der Atemschlauch - Anschlussvorrichtung mit der Atemvorrichtung verursachen und andererseits zu einer Undichtheit der Verbindung Atemvorrichtung / Patient führen. Der erfindungsgemässe Aufbau der Atemschlauch - Anschlussvorrichtung ermöglicht jedoch eine Positionierung des Transponders zwischen der Außenfläche des Grundkörpers und der Innenwandung des Hüllkörpers, so dass der Transponder von dem ihn umgebenden Hüllkörper der Atemschlauch - Anschlussvorrichtung vor Beschädigungen geschützt wird. Auf der Außenfläche des Grundkörpers sind vorzugsweise Hilfsmittel zur Positionierung des Transponders auf dem Grundkörper vorgesehen. Beispielsweise kann die an der Außenfläche des Grundkörpers vorgesehene Umfangswulst als Positionierungshilfe beim Aufbringen des Transponders auf den Grundkörper vorgesehen werden. Der Transponder ist bevorzugt an einer zu der Atemvorrichtung weisenden Seite des Grundkörpers angebracht. Damit können auch passive Transponder mit einer stark eingeschränkten Reichweite zum Einsatz kommen. Dies kann beispielsweise ein RFID Transponder sein, der eine Reichweite von zirka 18 Millimetern aufweist.

Der Transponder wird vorzugsweise auf die Außenfläche des Grundkörpers mittels eines geeigneten Klebeverfahrens aufgebracht. Klebeverfahren zum Aufbringen von Transpondern auf der Oberfläche eines bestimmten Teils sind aus dem Stand der Technik bekannt, daher wird an dieser Stelle nicht näher darauf eingegangen. Es kann jedoch auch in einem bevorzugten Verfahrensschritt kurz vor Beendigung oder nach der Herstellung des Grundkörpers durch ein Spritzgussverfahren vorgesehen sein, den Transponder auf den Grundkörper aufzubringen, indem der Grundkörper in einem Spritzgusswerkzeug vorzugsweise nach dessen Herstellung vorerst verbleibt und der Transponder mittels einer geeigneten Spritzgusstechnik auf die Außenfläche des Grundkörpers aufgebracht wird, ohne den Transponder dabei vollständig mit Spritzgussmaterial zu umgeben.

Der Transponder ermöglicht durch eine Speicherfunktion von Daten in vorteilhafter Weise eine Ablage von Atemparametern. Als Atemparameter können beispielsweise eine Atemfrequenz, ein Atemhub, ein Atemdruck und / oder eine Form der Beatmung hinterlegt werden. Auch sind Kenngrössen für eine Identifikation des Atemschlauches hinterlegbar. Der Atemschlauch verbleibt bei einem Wechsel der Atemvorrichtung, beispielsweise aufgrund eines Wechsels des klinischen Bereiches des Patienten, bei dem Patienten. Die in dem Transponder hinterlegten Daten sind somit mit dem Atemschlauch verfügbar und ermöglichen bei einem Anschluss des Atemschlauches an eine andere Atemvorrichtung eine automatische oder semiautomatische Einstellung der erforderlichen Atemparameter an dieser Atemvorrichtung. Somit lassen sich auf einfache Weise Einstellparameter einer ersten Atemvorrichtung auf eine zweite Atemvorrichtung übertragen. Des Weiteren können Daten über verbotene Parameter von weiteren Geräten in dem Transponder der Atemschlauch - Anschlussvorrichtung hinterlegt sein. Beispielsweise kann bei Anschluss des neonatalen Atemschlauches an eine Atemvorrichtung für Erwachsene nach einer Erkennung des neonatalen Atemschlauches ein Atemmodus für Erwachsene blockiert werden.

Darüber hinaus ermöglicht der Transponder der erfindungsgemässen Vorrichtung eine Erfassung und Ablage von personengebundenen Daten wie beispielsweise eine Patentenidentifikationsnummer. Mittels einer Patientenidentifikationsnummer kann sich eine, an ein Kliniknetzwerk angeschlossene Atemvorrichtung auf die für den entsprechenden Patienten erforderlichen Atemparameter einstellen.
In einer weiteren Gestaltung der erfindungsgemässen Atemschlauch - Anschlussvorrichtung werden Kenngrößen für eine Gewährleistung einer einwandfreien Funktion des Atemschlauches übertragen. Beispielsweise kann eine Lebensdauer des Atemschlauches auf dem Transponder hinterlegt werden und nach einem Zeitablauf, beispielsweise einer für eine Gewährleistung einer einwandfreien Funktion des Atemschlauches ermittelte und festgelegte Zeit, eine entsprechende Information dem Anwender übermittelt werden.

Ausführungsbeispiele der Erfindung sind in den Figuren 1 bis 4 beschrieben.

Dabei zeigen:
- Fig. 1: eine vereinfachte Schnittdarstellung der erfindungsgemässen Atemschlauch - Anschlussvorrichtung in einer ersten Ausführung,
- Fig. 2: eine vereinfachte Schnittdarstellung der erfindungsgemässen Atemschlauch - Anschlussvorrichtung zur Erläuterung einer Verbindung eines neonatalen Atemschlauches mit einer neonatalen Atemvorrichtung,
- Fig. 3: eine vereinfachte Schnittdarstellung der erfindungsgemässen Atemschlauch - Anschlussvorrichtung zur Erläuterung einer Verbindung eines neonatalen Atemschlauches mit einer Atemvorrichtung für Erwachsene und
- Fig. 4: eine vereinfachte Schnittdarstellung der erfindungsgemässen Atemschlauch - Anschlussvorrichtung in einer zweiten Ausführung mit einem Transponder.

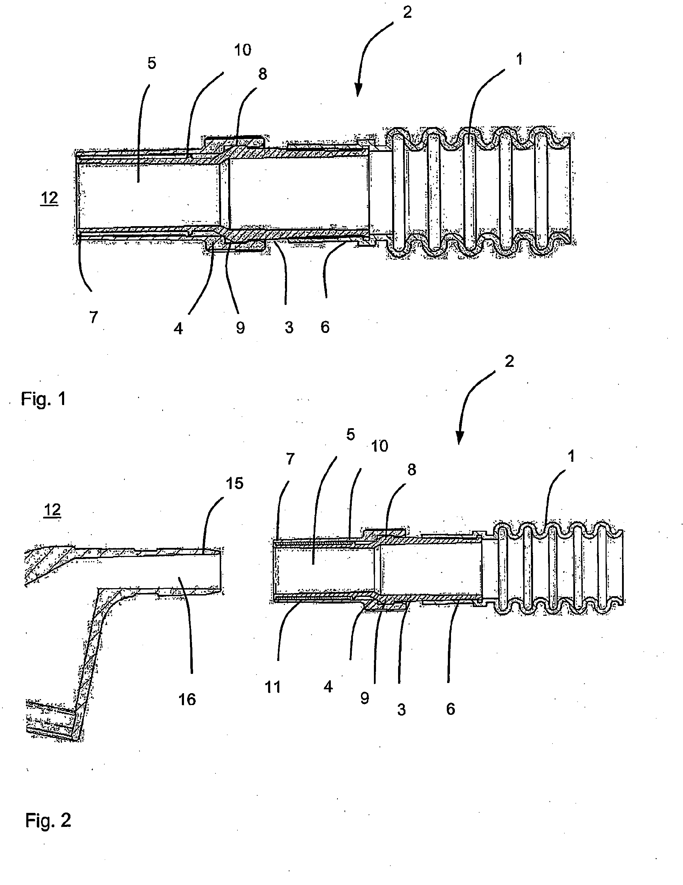

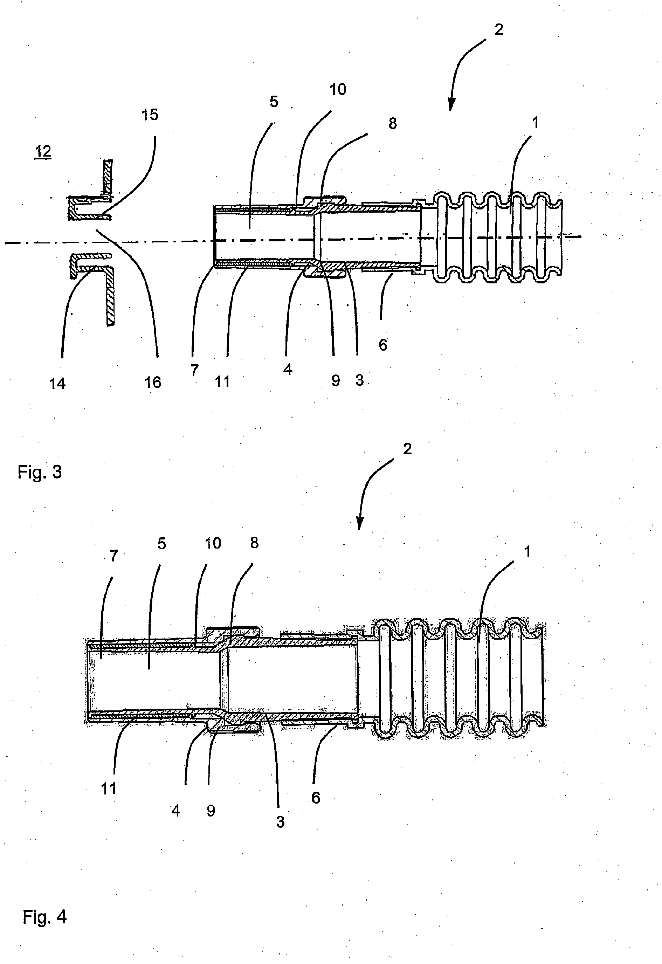

Der in Fig. 1 gezeigte Atemschlauch 1 ist in seinem Endbereich mit einer Atemschlauch - Anschlussvorrichtung 2 versehen, die einen Grundkörper 3 und einen zu einer Atemvorrichtung 12 gerichteten Teil des Grundkörpers 3 umschließenden Hüllkörper 4 aufweist. Im atemschlauchseitigen Bereich des Grundköpers 3 ist ein Atemschlauch - Befestigungsabschnitt 6 gebildet, in welchem der Atemschlauch 1 fixiert ist. Der Grundkörper 3 weist einen Atemgasdurchgangskanal 5 auf, dessen Durchgangsquerschnitt im Wesentlichen dem Durchgangsquerschnitt des an den Atemschlauch - Befestigungsabschnitt 6 angeschlossenen Atemschlauches 1 entspricht. Sowohl die Außenfläche des Grundkörpers 3 als auch die Innenwandung des Hüllkörpers 4 der in Fig. 1 dargestellte Atemschlauch - Anschlussvorrichtung 2 sind zylindrisch ausgebildet. In einem mittleren Bereich des Grundkörpers 3 ist an dessen Außenseite eine Umfangswulst 8 vorgesehen, über die ein unter mechanischen Gesichtspunkten günstiger Formschluss mit einer komplementären, an der Innenwandung des Hüllkörpers 4 befindlichen Umfangsnut 9, erreicht wird. Um ein Verdrehen des Hüllkörpers 4 gegenüber dem Grundkörper 3 bei einem Verbinden oder Lösen der Atemschlauch - Anschlussvorrichtung 2 mit oder von einer Atemvorrichtung 12 zu verhindern, ist eine senkrecht zu einer Außenfläche des Grundkörpers 3 gerichtete Nase vorgesehen, die in eine senkrecht zu der Außenseite des Hüllkörpers 4 gerichtete komplementäre Aussparung eingreift. Ein Einrasten der Nase des Grundkörpers 3 in die Aussparung des Hüllkörpers 4 signalisiert dabei eine exakte Verbindung von Grundkörper 3 und Hüllkörper 4. Der Grundkörper 3 und der Hüllkörper 4 sind vorzugsweise, aufgrund der guten elastischen Eigenschaften, aus Polypropylen (PP) ausgeführt. Es ist aber auch möglich, den Grundkörper 3 und Hüllkörper 4 aus anderen geeigneten Materialien zu fertigen. In den Figuren 2 und 3 sind zum Zwecke der Erläuterung der Verbindung von der erfindungsgemässen Atemschlauch - Anschlussvorrichtung 2 mit einer Atemvorrichtung 12 eine atemvorrichtungsseitige Anschlussstruktur 16 dargestellt, die im Wesentlichen komplementär zu dem von dem Grundkörper 3 und dem Hüllkörper 4 ausgebildeten Kopplungsabschnitt 7 ausgebildet ist. Dabei zeigt Fig. 2 eine Atemschlauch - Anschlussvorrichtung 2 zur Verbindung mit einer neonatalen Atemvorrichtung 12. Eine neonatale Atemvorrichtung 12 ist im Allgemeinen mit einer männlichen Anschlussstruktur 16 ausgeführt, die einen Durchmesser von 11 Millimetern aufweist. Eine Atemvorrichtung 12 für Erwachsene, wie sie in Fig. 3 dargestellt ist, weist hingegen üblicherweise eine männliche und eine weibliche Anschlussstruktur 16 auf, wobei die männliche Anschlussstruktur 16 einen Durchmesser von 15 Millimetern und die weibliche Anschlussstruktur 16 einen Durchmesser von 11 Millimetern besitzt. Die erfindungsgemässe Atemschlauch - Anschlussvorrichtung 2 kann in vorteilhafter Weise eine Verbindung eines neonatalen Atemschlauches 1 mit einer neonatale Atemvorrichtung 12 als auch mit einer Atemvorrichtung 12 für Erwachsene realisieren. Bei der Verbindung der Atemschlauch - Anschlussvorrichtung 2 eines neonatalen Atemschlauches 1 an eine in Fig. 2 schematisch dargestellte Anschlussstruktur 16 einer neonatalen Atemvorrichtung 12 gelangt der durch das Bezugszeichen 7 gekennzeichnete Kopplungsabschnitt der Atemschlauch - Anschlussvorrichtung 2 mit einem Zapfenabschnitt 15 der Anschlussstruktur 16 der neonatalen Atemvorrichtung 12 in Eingriff. Der Kopplungsabschnitt 7 wird von der Innenwandung des Grundkörpers 3, dem Atemgasdurchgangskanal 5, gebildet. Ein zu der Atemvorrichtung 12 gerichteter Teil des Atemgasdurchgangskanals 5 des Grundkörpers 3 ist in vorteilhafter Weise konusförmig ausgebildet. Der Zapfenabschnitt 15 der Anschlussstruktur 16, der in Fig. 2 dargestellten neonatalen Atemvorrichtung 12 weist eine dem Koppelabschnitt 7 komplementäre Form auf.

Bei der Verbindung der Atemschlauch - Anschlussvorrichtung 2 eines neonatalen Atemschlauches 1 an eine, in Fig. 3 schematisch dargestellte, Anschlussstruktur 16 der Atemvorrichtung 12 für Erwachsene, gelangt der Kopplungsabschnitt 7 der Atemschlauch - Anschlussvorrichtung 2 mit einer Ausnehmung 14 und dem Zapfenabschnitt 15 der Anschlussstruktur 16 der Atemvorrichtung 12 für Erwachsene in Eingriff. Der Kopplungsabschnitt 7 wird von der Innenwandung des Grundkörpers 3 und der Außenfläche des Hüllkörpers 4 gebildet. Somit lässt sich mit der erfindungsgemässen Atemschlauch - Anschlussvorrichtung 2 ein neonataler Atemschlauch 1 sowohl mit der Anschlussstruktur 16 der neonatalen Atemvorrichtung 12 (dargestellt in Fig. 2) als auch mit der Anschlussstruktur 16 einer Atemvorrichtung 12 für Erwachsene (dargestellt in Fig. 3) verbinden. Die Außenfläche des Hüllkörpers 4 ist, wie in Fig. 3 gezeigt, in vorteilhafter Weise konusförmig ausgebildet und wirkt mit der konusförmigen Ausnehmung 14 der Anschlussstruktur 16 der Atemvorrichtung 12 für Erwachsene zusammen.

In Fig. 4 ist eine ganz besonders bevorzugte Ausführung der Atemschlauch - Anschlussvorrichtung 2 gezeigt. Zwischen der Außenfläche des Grundkörpers 3 und der Innenwandung des Hüllkörpers 4 ist ein Transponder 11 vorgesehen. Der Transponder 11 dient der drahtlosen Sendung und des drahtlosen Empfangs von Daten und ist zudem mit einer Funktion zur Speicherung der Daten ausgestattet. Die auf der Außenfläche des Grundkörpers 3 vorgesehene Umfangswulst 8 dient als Positionierungshilfe beim Aufbringen des Transponders 11 auf dem Grundkörper 3. Damit wird gewährleistet, dass der Transponder 11 eine reproduzierbare Position aufweist und sehr nah an einem Ende einer zu der Atemvorrichtung 12 weisenden Seite des Grundkörpers 3 vorgesehen ist. Dies ermöglicht die Verwendung von passiven Transpondem 11, beispielsweise eines RFID - Transponders 11 mit einer Reichweite von zirka 18 Millimetern. Der Empfang und die Sendung von Daten sind somit optimal gewährleistet. Der Transponder 11 der erfindungsgemässen Atemschlauch - Anschlussvorrichtung 2 der Fig. 4 ermöglicht durch eine Speicherfunktion von Daten in vorteilhafter Weise eine Ablage von Atemparametem, wie beispielsweise eine Atemfrequenz, einen Atemhub, einen Atemdruck und / oder eine Form der Beatmung. Wird nur der Atemschlauch 1 von einer neonatale Atemvorrichtung 12 entkoppelt und an eine Atemvorrichtung 12 für Erwachsene gekoppelt, können die auf dem Transponder 11 hinterlegten Atemparameter der neonatalen Atemvorrichtung 12 auf die Atemvorrichtung 12 für Erwachsene übertragen werden, bzw. diese Atemparameter an der Atemvorrichtung 12 für Erwachsene eingestellt werden. Zudem kann ein Atemmode für Erwachsene gesperrt und nur ein Atemmode für Neonaten an der Atemvorrichtung 12 für Erwachsene freigegeben werden. Damit ist eine Einstellung von zu hohen Atemdrücken für Neonaten, wie sie für eine Beatmung von Erwachsenen erforderlich sind, ausgeschlossen. Weiterhin lassen sich personengebundene Daten, wie beispielsweise eine Patentenidentifikationsnummer, mit dem Transponder 11 der erfindungsgemässen Atemschlauch - Anschlussvorrichtung 2 speichern. Der Atemschlauch 1 verbleibt bei einem Wechsel des klinischen Bereiches und damit der Atemvorrichtung am Patienten. Die in dem Transponder 11 hinterlegten Daten sind somit mit dem Atemschlauch 1 verfügbar und ermöglichen bei einem Anschluss des Atemschlauches 1 an die Atemvorrichtung 12 für Erwachsene eine Übertragung der Daten an die Atemvorrichtung 12. Mittels einer Patientenidentifikationsnummer kann sich beispielsweise eine an ein Kliniknetzwerk angeschlossene Atemvorrichtung 12 auf die für den entsprechenden Patienten erforderlichen Atemparameter einstellen.

In einer weiteren Ausgestaltung der in der Fig. 4 gezeigten Atemschlauch-Anschlussvorrichtung 2 werden Kenngrössen für eine Gewährleistung einer einwandfreien Funktion des Atemschlauches 1 auf den Transponder 11 übertragen. Beispielsweise wird die Zeit der Nutzung des Atemschlauches 1 an der jeweiligen Atemvorrichtung 12 gemessen und jeweils auf den Transponder 11 übermittelt. Somit kann die gesamte Lebensdauer des Atemschlauches 1 auf dem Transponder 11 hinterlegt werden und nach dem Erreichen einer Zeitdauer, die einen Ablauf für die Gewährleistung der einwandfreien Funktion des Atemschlauches 1 definiert, kann eine entsprechende Information dem Anwender übermittelt werden.

Ein bevorzugtes Verfahren zur Herstellung der erfindungsgemäßen Atemschlauch - Anschlussvorrichtung 2 wird im Folgenden anhand des in Fig. 4 dargestellten Ausführungsbeispiels erläutert. In einem ersten Verfahrensschritt wird der Grundkörper 3 mittels eines Spritzgussverfahrens hergestellt. Der Grundkörper 3 weist dabei eine von der Außenfläche herausragende Umlaufwulst 8 auf, die zur Positionierung eines in einem weiteren Verfahrensschritt auf die Außenfläche des Grundkörpers 3 aufzubringenden Transponders 11 dient. Der Transponder 11 wird dabei auf einem zu der Atemvorrichtung 12 weisenden Teil der Außenfläche des Grundkörpers 3 aufgebracht. Danach oder parallel wird der Hüllkörper 4 mittels eines Spritzgussverfahrens hergestellt. In einem weiteren Verfahrensschritt wird der Grundkörper 3 mit dem Hüllkörper 4 formschlüssig verbunden.

### BEZUGSZEICHENLISTE

- 1: Atemschlauch
- 2: Atemschlauch - Anschlussvorrichtung
- 3: Grundkörper
- 4: Hüllkörper
- 5: Atemgasdurchgangskanal
- 6: Atemschlauch - Befestigungsabschnitt
- 7: Kopplungsabschnitt
- 8: Umfangswulst
- 9: Umfangsnut
- 10: Verdrehsicherung
- 11: Transponder
- 12: Atemvorrichtung
- 14: Ausnehmung
- 15: Zapfenabschnitt
- 16: Anschlussstruktur

## Patentansprüche

1. Atemschlauch - Anschlussvorrichtung zur Verbindung eines Atemschlauches mit einer Atemvorrichtung bestehend aus
- einem Grundkörper (3) mit einem in dem Grundkörper (3) ausgebildeten Atemgasdurchgangskanal (5) und
- einem Hüllkörper (4), welcher mit dem Grundkörper (3) formschlüssig in Eingriff bringbar ist und dabei zumindest einen Teil des Grundkörpers (3) koaxialförmig umschließt, **dadurch gekennzeichnet, dass**
ein Transponder (11) zwischen dem Grundkörper (3) und dem Hüllkörper (4) vorgesehen ist.

2. Atemschlauch - Anschlussvorrichtung nach Anspruch 1, **dadurch gekennzeichnet, dass** der Transponder (11) den Grundkörper (3) koaxialförmig umschließt.

3. Atemschlauch - Anschlussvorrichtung nach einem der Ansprüche 1 oder 2, **dadurch gekennzeichnet, dass** die Außenfläche des Grundkörpers (3) Hilfsmittel zur Positionierung des Transponders (11) auf dem Grundkörper (3) aufweist.

4. Atemschlauch - Anschlussvorrichtung nach einem der vorhergehenden Ansprüche, **dadurch gekennzeichnet, dass** der Transponder (11) ein Speicherelement enthält.

5. Atemschlauch - Anschlussvorrichtung nach einem der vorhergehenden Ansprüche, **dadurch gekennzeichnet, dass** der Transponder (11) an einer zu der Atemvorrichtung (12) weisenden Seite des Grundkörpers (3) vorgesehen ist.

6. Atemschlauch - Anschlussvorrichtung nach einem der vorhergehenden Ansprüche, **dadurch gekennzeichnet, dass** der Durchgangsquerschnitt des Atemgasdurchgangskanals (5) im Wesentlichen dem Durchgangsquerschnitt des Atemschlauchs (1) entspricht.

7. Atemschlauch - Anschlussvorrichtung nach einem der vorhergehenden Ansprüche, **dadurch gekennzeichnet, dass** ein zu der Atemvorrichtung (12) gerichtete Teil des Atemgasdurchgangskanals (5) konusförmig verläuft und eine Außenfläche des Grundkörpers (3) zylindrisch ausgebildet ist.

8. Atemschlauch - Anschlussvorrichtung nach einem der vorhergehenden Ansprüche, **dadurch gekennzeichnet, dass** eine Innenwandung des Hüllkörpers (4) zylindrisch ausgebildet ist und eine Außenfläche konisch verläuft.

9. Atemschlauch - Anschlussvorrichtung nach einem der vorhergehenden Ansprüche, **dadurch gekennzeichnet, dass** ein Formschluss von Grund- und Hüllkörper (3, 4) durch einen Eingriff einer an der Außenfläche des Grundkörpers (3) vorgesehenen Umfangswulst (8) in eine komplementäre an der Innenwandung des Hüllkörpers (4) vorgesehenen Umfangsnut (9) erfolgt.

10. Atemschlauch - Anschlussvorrichtung nach einem der vorhergehenden Ansprüche, **dadurch gekennzeichnet, dass** Mittel (10) vorgesehen sind, die ein Verdrehen des Hüllkörpers (4) in Bezug zu dem Grundkörper (3) verhindern.

11. Atemschlauch - Anschlussvorrichtung nach Anspruch 10, **dadurch gekennzeichnet, dass** der Grundkörper (3) eine senkrecht zu der Außenfläche gerichtete Nase und der Hüllkörper (4) eine senkrecht zu der Innenwandung gerichtete komplementäre Aussparung aufweist.

12. Atemschlauch - Anschlussvorrichtung nach einem der vorhergehenden Ansprüche, **dadurch gekennzeichnet, dass** der Grundkörper (3) und der Hüllkörper (4) aus Polypropylen besteht.

13. Verwendung einer Atemschlauch - Anschlussvorrichtung nach einem der vorhergehenden Ansprüche zur Verbindung eines Atemschlauchs (1) mit einer ersten oder einer zweiten Atemvorrichtung (12), wobei die erste und die zweite Atemvorrichtung (12) eine unterschiedliche Anschlussstruktur (16) aufweist.

14. Verwendung einer Atemschlauch - Anschlussvorrichtung nach einem der vorhergehenden Ansprüche, **dadurch gekennzeichnet, dass** Parameter der ersten Atemvorrichtung (12) auf die zweite Atemvorrichtung (12) übertragbar sind.

## Claims

1. A respiratory-hose connecting device for connecting a respiratory hose to a respiratory device consisting of
- a base body (3) having a respiratory-gas through-channel (5) formed in the base body (3), and
- an enveloping body (4) which can be engaged with the base body (3) in a form-locking manner and thereby coaxially encloses at least a portion of the base body (3), **characterised in that**
a transponder (11) is provided between the base body (3) and the enveloping body (4).

2. A respiratory-hose connecting device according to claim 1, **characterised in that** the transponder (11) coaxially encloses the base body (3).

3. A respiratory-hose connecting device according to one of claims 1 or 2, **characterised in that** the outer face of the base body (3) has auxiliary means to position the transponder (11) on the base body (3).

4. A respiratory-hose connecting device according to one of the preceding claims, **characterised in that** the transponder (11) contains a storage element.

5. A respiratory-hose connecting device according to one of the preceding claims, **characterised in that** the transponder (11) is provided on a side of the base body (3) that points to the respiratory device (12).

6. A respiratory-hose connecting device according to one of the preceding claims, **characterised in that** the opening cross-section of the respiratory-gas through-channel (5) substantially corresponds to the opening cross-section of the respiratory hose (1).

7. A respiratory-hose connecting device according to one of the preceding claims, **characterised in that** a portion of the respiratory-gas through-channel (5) that is directed to the respiratory device (12) extends in a conical manner, and an outer face of the base body (3) is formed cylindrically.

8. A respiratory-hose connecting device according to one of the preceding claims, **characterised in that** an inner wall of the enveloping body (4) is formed cylindrically, and an outer face extends conically.

9. A respiratory-hose connecting device according to one of the preceding claims, **characterised in that** a form-lock of the base body (3) and the enveloping body (4) is effected by means of engagement of a circumferential bead (8) provided on the outer face of the base body (3) into a complementary circumferential groove (9) provided on the inner wall of the enveloping body (4).

10. A respiratory-hose connecting device according to one of the preceding claims, **characterised in that** means (10) are provided that prevent rotation of the enveloping body (4) in relation to the base body (3).

11. A respiratory-hose connecting device according to claim 10, **characterised in that** the base body (3) has a lug directed perpendicularly to the outer face, and the enveloping body (4) has a complementary recess that is directed perpendicularly to the inner wall.

12. A respiratory-hose connecting device according to one of the preceding claims, **characterised in that** the base body (3) and the enveloping body (4) consist of polypropylene.

13. Use of a respiratory-hose connecting device according to one of the preceding claims for connecting a respiratory hose (1) to a first or a second respiratory device (12), wherein the first and the second respiratory device (12) have different connecting structures (16).

14. Use of a respiratory-hose connecting device according to one of the preceding claims, **characterised in that** parameters of the first respiratory device (12) can be transferred to the second respiratory device (12).

## Revendications

1. Dispositif de raccordement de tuyau respiratoire, conçu pour relier un tuyau respiratoire à un appareil respiratoire et comprenant
- un corps de base (3) muni d'un canal (5) de passage d'un gaz respiratoire, pratiqué dans ledit corps de base (3), et
- un corps d'enveloppement (4) qui peut être mis en prise avec le corps de base (3) par complémentarité de formes, et qui ceinture alors au moins une partie dudit corps de base (3), de manière coaxiale, **caractérisé par le fait**
**qu'**un transpondeur (11) est prévu entre le corps de base (3) et le corps d'enveloppement (4).

2. Dispositif de raccordement de tuyau respiratoire, selon la revendication 1, **caractérisé par le fait que** le transpondeur (11) ceinture le corps de base (3) de manière coaxiale.

3. Dispositif de raccordement de tuyau respiratoire, selon l'une des revendications 1 ou 2, **caractérisé par le fait que** la face extérieure du corps de base (3) comporte des moyens auxiliaires affectés au positionnement du transpondeur (11) sur ledit corps de base (3).

4. Dispositif de raccordement de tuyau respiratoire, selon l'une des revendications précédentes, **caractérisé par le fait que** le transpondeur (11) comprend un élément de mémorisation.

5. Dispositif de raccordement de tuyau respiratoire, selon l'une des revendications précédentes, **caractérisé par le fait que** le transpondeur (11) est prévu sur un côté du corps de base (3) divisé vers l'appareil respiratoire (12).

6. Dispositif de raccordement de tuyau respiratoire, selon l'une des revendications précédentes, **caractérisé par le fait que** la section transversale de passage du canal (5) de passage du gaz respiratoire correspond, pour l'essentiel, à la section transversale de passage du tuyau respiratoire (1).

7. Dispositif de raccordement de tuyau respiratoire, selon l'une des revendications précédentes, **caractérisé par le fait qu'**une partie du canal (5) de passage du gaz respiratoire, dirigée vers l'appareil respiratoire (12), présente une configuration conique, une face extérieure du corps de base (3) étant de réalisation cylindrique.

8. Dispositif de raccordement de tuyau respiratoire, selon l'une des revendications précédentes, **caractérisé par le fait qu'**une paroi intérieure du corps d'enveloppement (4) est de réalisation cylindrique, une face extérieure présentant une configuration conique.

9. Dispositif de raccordement de tuyau respiratoire, selon l'une des revendications précédentes, **caractérisé par le fait qu'**un assemblage des corps (3, 4) de base et d'enveloppement, par complémentarité de formes, est instauré par pénétration d'un bourrelet périphérique (8), prévu à la face extérieure dudit corps de base (3), dans une rainure périphérique complémentaire (9) prévue dans la paroi intérieure dudit corps d'enveloppement (4).

10. Dispositif de raccordement de tuyau respiratoire, selon l'une des revendications précédentes, **caractérisé par** la présence de moyens (10) prévenant une rotation du corps d'enveloppement (4) vis-à-vis du corps de base (3).

11. Dispositif de raccordement de tuyau respiratoire, selon la revendication 10, **caractérisé par le fait que** le corps de base (3) comporte un mentonnet ou nez dirigé perpendiculairement à la face extérieure, et le corps d'enveloppement (4) présente un évidement complémentaire dirigé perpendiculairement à la paroi intérieure.

12. Dispositif de raccordement de tuyau respiratoire, selon l'une des revendications précédentes, **caractérisé par le fait que** le corps de base (3) et le corps d'enveloppement (4) sont réalisés en polypropylène.

13. Utilisation d'un dispositif de raccordement de tuyau respiratoire, conforme à l'une des revendications précédentes, pour relier un tuyau respiratoire (1) à un premier ou à un second appareil respiratoire (12), lesdits premier et second appareils respiratoires (12) présentant une structure de raccordement (16) différente.

14. Utilisation d'un dispositif de raccordement de tuyau respiratoire, conforme à l'une des revendications précédentes, **caractérisée par le fait que** des paramètres du premier appareil respiratoire (12) peuvent être transposés au second appareil respiratoire (12).
